(19) Europäisches Patentamt
European Patent Office
Office européen des brevets

(11) Veröffentlichungsnummer : **0 062 181**
**B1**

(12) # EUROPÄISCHE PATENTSCHRIFT

(45) Veröffentlichungstag der Patentschrift : **05.06.85**

(51) Int. Cl.[4] : **A 01 N 25/04**, A 01 N 25/30, A 61 K 9/10

(21) Anmeldenummer : **82102073.2**

(22) Anmeldetag : **15.03.82**

(54) **Öl-in-Wasser-Emulsionen, Verfahren zu deren Herstellung und deren Verwendung.**

(30) Priorität : **26.03.81 DE 3111934**

(43) Veröffentlichungstag der Anmeldung : **13.10.82 Patentblatt 82/41**

(45) Bekanntmachung des Hinweises auf die Patenterteilung : **05.06.85 Patentblatt 85/23**

(84) Benannte Vertragsstaaten : **AT BE CH DE FR GB IT LI NL SE**

(56) Entgegenhaltungen :
**EP-A- 0 055 401**
**CH-A- 275 705**
**DE-A- 2 041 480**
**DE-B- 1 121 814**
**DE-C- 824 949**
**FR-A- 2 452 250**

(73) Patentinhaber : **BAYER AG**
**Konzernverwaltung RP Patentabteilung**
**D-5090 Leverkusen 1 Bayerwerk (DE)**

(72) Erfinder : **Hausmann, Heinz, Dr.**
**Dierath 5**
**D-5653 Leichlingen 1 (DE)**
Erfinder : **Niessen, Heinz Josef, Dr.**
**Pannenberg 73**
**D-5060 Bergisch Gladbach 2 (DE)**
Erfinder : **Telle, Otto**
**Haferkamp 10**
**D-5000 Köln 80 (DE)**
Erfinder : **Neumaier, Hermann, Dr.**
**Kurlandweg 33**
**D-5653 Leichlingen 1 (DE)**

**EP 0 062 181 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Öl-in-Wasser-Emulsionen von agrochemischen Wirkstoffen, Wirkstoffen zur Bekämpfung von Schädlingen im Haushalts- und Hygiene-Bereich und/oder pharmakologisch wirksamen Stoffen. Die Erfindung betrifft außerdem ein Verfahren zur Herstellung dieser Öl-in-Wasser-Emulsionen sowie deren Verwendung.

Es sind bereits Öl-in-Wasser-Emulsionen von zahlreichen in Wasser wenig löslichen agrochemischen Wirkstoffen bekannt, welche neben den Wirkstoffen jeweils noch entweder einen oberflächenaktiven Stoff und ein Verdickungsmittel oder aber eine relativ große Menge an oberflächenaktiven Stoffen enthalten (vgl. DE-A 30 09 944, DE-A 30 11 611, FR-A 2 452 250 und JP-A 122 628-77). Dieser Zusatz an Verdickungsmittel bzw. an großen Tensidmengen ist mit zusätzlichen Kosten verbunden und stellt somit einen gravierenden Nachteil der bekannten Öl-in-Wasser-Emulsionen dar. Hinzu kommt, daß die bisher beschriebene Herstellung von derartigen Emulsionen nicht allgemein anwendbar ist. Im wesentlichen lassen sich danach nämlich nur solche in Wasser wenig löslichen Wirkstoffe emulgieren, die bei Raumtemperatur flüssig sind oder zumindest einen sehr niedrigen Schmelzpunkt aufweisen. Nachteilig ist auch, daß die bekanten Öl-in-Wasser-Emulsionen häufig nicht ausreichend kältestabil sind, und daß in manchen Fällen eine Zwangsemulgierung mit Hilfe von Homogenisatoren erforderlich ist.

Weiterhin sind aus der DE-C 824 949, der DE-B 1 121 814, der CH-A 275 705 und der EP-A 0 055 401 zahlreiche Alkyl-aryl-polyglykolether und deren Verwendung als Emulgatoren in agrochemischen Mitteln bekannt. Kombinationen dieser nicht-ionischen Emulgatoren mit ionischen Komponenten werden in den genannten Druckschriften allerdings nicht erwähnt.

Ferner geht aus der DE-A 2 041 480 hervor, daß Emulgator-Kombinationen aus einer ionischen und einer nicht-ionischen Komponente zur Herstellung von Emulsionen geeignet sind, welche agrochemische Wirkstoffe enthalten.

Die betreffenden Emulsionen enthalten jedoch relativ große Mengen an organischen Solventien und sind nur relativ kurze Zeit beständig.

Es wurden nun Öl-in-Wasser-Emulsionen gefunden, die

— 5 bis 80 Gew.-% mindestens eines in Wasser wenig löslichen agrochemischen Wirkstoffes, eines Wirkstoffes zur Bekämpfung von Schädlingen im Haushalts- und Hygiene-Bereich und/oder eines pharmakologisch wirksamen Stoffes,

— 3 bis 15 Gew.-% mindestens eines Alkylaryl-polyglykolethers der Formel

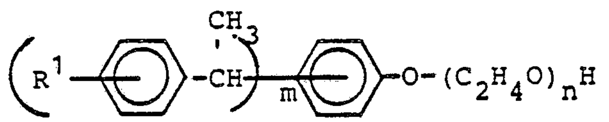

(I)

in welcher

$R^1$ für Wasserstoff oder Methyl steht,

m für 2 oder 3 steht und

n für ganze Zahlen von 12 bis 40 steht,

im Gemisch mit Alkylarylsulfonsäure-Salzen der Formel

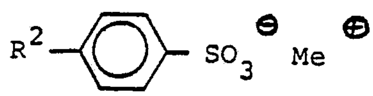

(II)

in welcher

$R^2$ für Alkyl mit 9 bis 30 Kohlenstoffatomen steht und

$Me^{\oplus}$ für ein Natriumkation oder für ein Äquivalent eines Calciumkations steht,

— und Wasser, und

— gegebenenfalls 5 bis 20 Gew.-% mindestens eines mit Wasser wenig mischbaren organischen Lösungsmittels und/oder eines Solubilisators sowie

— gegebenenfalls 0,1 bis 10 Gew.-% an Zusatzstoffen

wobei die Summe der Komponenten jeweils 100 Gew.-% beträgt.

Weiterhin wurde gefunden, daß sich die erfindungsgemäßen Öl-in-Wasser-Emulsionen dadurch herstellen lassen, daß man eine homogene Mischung aus

— mindestens einem in Wasser wenig löslichen agrochemischen Wirkstoff, einem Wirkstoff zur Bekämpfung von Schädlingen im Haushalts- und Hygiene-Bereich und/oder einen pharmakologisch wirksamen Stoff,

— mindestens einem Alkylaryl-polyglykolether der Formel

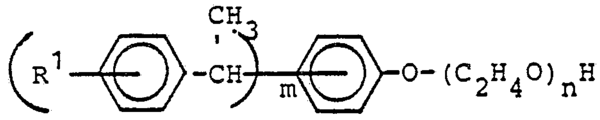

(I)

in welcher
$R^1$, m und n die oben angegebene Bedeutung haben,
im Gemisch mit Alkylaryl-sulfonsäure-Salzen der Formel

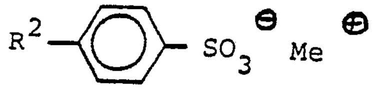

(II)

in welcher
$R^2$ und $Me^{\oplus}$ die oben angegebene Bedeutung haben,
— gegebenenfalls mindestens einem mit Wasser wenig mischbaren organischen Lösungsmittel und/oder einem Solubilisator sowie
— gegebenenfalls Zusatzstoffen
unter Rühren in gegebenenfalls Zusatzstoffe enthaltendes Wasser gibt.

Schließlich wurde gefunden, daß sich die erfindungsgemäßen Öl-in-Wasser-Emulsionen, — je nach den enthaltenen Wirkstoffen—, für verschiedene Zwecke in der Landwirtschaft und im Gartenbau, im Haushalts- und Hygiene-Bereich oder im medizinischen Bereich verwenden lassen.

Es ist als äußerst überraschend zu bezeichnen, daß die erfindungsgemäßen Öl-in-Wasser-Emulsionen stabil sind, denn auf Grund des bekannten Standes der Technik war zu erwarten, daß derartige Emulsionen, die keine Verdickungsmittel und auch nur einen geringen Tensidanteil aufweisen, nicht über längere Zeit haltbar sind. So geht aus der DE-A 30 09 944 und der DE-A 30 11 611 bzw. der FR-A 2 452 250 hervor, daß die dort beschriebenen Öl-in-Wasser-Emulsionen zwingend ein Verdickungsmittel als Stabilisator enthalten. Die in der JP-A 122 628-77 offenbarten Emulsionen weisen einen im Verhältnis zur Wirkstoffmenge sehr hohen Tensidanteil auf. Die hervorragende Beständigkeit der erfindungsgemäßen Öl-in-Wasser-Emulsionen konnte daher nicht vorausgesehen werden.

Ferner mußte unter Berücksichtigung der technischen Lehre, die aus der DE-A 2 041 480 hervorgeht, angenommen werden, daß auch Emulgator-Kombinationen aus einer ionischen und einer nicht-ionischen Komponente nur dann zur Herstellung von stabilen wäßrigen Emulsionen geeignet sind, wenn eine relativ große Menge an organischem Lösungsmittel vorhanden ist. Im Gegensatz zu den Erwartungen zeigte sich jedoch, daß sich die erfindungsgemäßen Emulsionen durch eine hervorragende Beständigkeit auszeichnen, obwohl sie keine oder nur geringe Mengen an organischen Lösungsmitteln enthalten.

Die erfindungsgemäßen Öl-in-Wasser-Emulsionen zeichnen sich durch eine Reihe von Vorteilen aus. So erübrigt sich bei ihrer Herstellung ein kostspieliger Zusatz von Verdickungsmitteln oder großen Mengen an Emulgatoren.

Weiterhin enthalten diese Emulsionen entweder gar keine, oder aber nur eine extrem geringe Menge an organischen Lösungsmitteln. Sie sind daher nicht brennbar, frei oder zumindest nahezu frei von Geruchsbelästigungen durch organische Lösungsmittel und weisen eine geringere Toxizität bzw. Phytotoxizität auf als entsprechende Formulierungen, die organische Lösungsmittel in den sonst üblichen Konzentrationen enthalten. Darüber hinaus sind die erfindungsgemäßen Öl-in-Wasser-Emulsionen unter den in der Praxis herrschenden Bedingungen stabil. Bei Langzeit-Lagerungen bleiben diese Emulsionen sowohl bei Temperaturen von 50 °C als auch bei tiefen Temperaturen unverändert. Schließlich lassen sich die erfindungsgemäßen Öl-in-Wasser-Emulsionen in einfacher Weise herstellen. Eine Zwangsemulgierung mit Hilfe von Homogenisatoren ist nicht erforderlich. Außerdem besteht ein ganz erheblicher Vorteil darin, daß sich bei Raumtemperatur feste bzw. flüssige, in Wasser wenig lösliche Wirkstoffe gleichermaßen gut emulgieren lassen.

Die erfindungsgemäßen Öl-in-Wasser-Emulsionen enthalten mindestens einen in Wasser wenig löslichen agrochemischen Wirkstoff, einen Wirkstoff zur Bekämpfung von Schädlingen im Haushalts- und Hygiene-Bereich und/oder einen pharmakologisch wirksamen Stoff. Diese Wirkstoffe liegen in der Ölphase im flüssigen Zustand vor.

Als Wirkstoffe kommen sowohl solche Substanzen in Frage, die bei Raumtemperatur flüssig sind, als auch solche, die bei Raumtemperatur fest sind. Voraussetzung für flüssige Wirkstoffe ist lediglich, daß sie in Wasser schwer löslich sind. Unter derartigen Stoffen sind hierbei Substanzen zu verstehen, die in Wasser bei 20 °C zu maximal 0,5 Gew.-% löslich sind. Feste Wirkstoffe müssen darüber hinaus jedoch in einem mit Wasser wenig mischbaren organischen Lösungsmittel und/oder einem Solubilisator hinreichend löslich sein.

Unter agrochemischen Stoffen sind im vorliegenden Fall alle üblicherweise im Pflanzenschutz verwendbaren Wirkstoffe zu verstehen. Hierzu gehören zum Beispiel Insektizide, Akarizide, Nematizide, Fungizide, Herbizide, Wachstums regulatoren und Düngemittel. Als Beispiele für derartige Wirkstoffe seien im einzelnen genannt :

O,O-Diethyl-O-(4-nitro-phenyl)-thiono-phosphorsäureester
O,O-Dimethyl-O-(4-nitro-phenyl)-thiono-phosphorsäureester
O-(Ethyl-O-(4-methylthio-phenyl)-S-propyl-dithiophosphat
(O,O-Diethylthionophosphoryl)-α-oximino-phenylessigsäurenitril

2-Isopropoxy-phenyl-N-methylcarbamat
3-Methylthio-4-amino-6-tert.-butyl-1,2,4-triazin-5-on
3-Methylthio-4-isobutylidenamino-6-tert.-butyl-1,2,4-triazin-5-on
2-Chlor-4-ethylamino-6-isopropylamino-1,3,5-triazin
2,3-Dihydro-2,2-dimethyl-7-benzofuranyl-methyl-carbamat
3,5-Dimethyl-4-methylthiophenyl-N-methyl-carbamat
O,O-Diethyl-O-(3-chlor-4-methyl-7-cumarinyl)-thiophosphat
γ-Hexachlorcyclohexan
6,7,8,9,10,10-Hexachlor-1,5,5A,6,9,9A-hexahydro-6,9-methan-2,4,3-benzo-dioxathiepin-3-oxid
1,4,5,6,7,8,8-Heptachlor-4,7-endo-methylen-3A,4,7,7A-tetrahydroinden
2-(2-Furyl)-benzimidazol
5-Amino-1-bis-(dimethylamido)-phosphoryl-3-phenyl-1,2,4-triazol
4-Hydroxy-3-(1,2,3,4-tetrahydro-1-naphthyl)-cumarin
S-[1,2-Bis-(ethoxycarbonyl)-ethyl]-O,O-dimethyl-dithiophosphorsäureester
O,O-Dimethyl-O-(4-methylmercapto-3-methyl-phenyl)-thionophosphorsäureester
O-Ethyl-O-(2-isopropyloxycarbonyl-phenyl)-N-isopropylthionophosphorsäureesteramid
1-(4-Chlorphenoxy)-3,3-dimethyl-1-(1,2,4-triazol-1-yl)-2-butanon
(S)-α-Cyano-3-phenoxybenzyl (1R)-cis-3-(2,2-dibromvinyl)-2,2-dimethylcyclopropancarboxylat
2,2-Dimethyl-3-(β,β-dichlorvinyl)-cyclopropancarbonsäure-α-cyano-3-phenoxy-4-fluor-benzylester

Unter Wirkstoffen zur Bekämpfung von Schädlingen im Haushalts- und Hygiene-Bereich sind im vorliegenden Fall alle üblichen in Wasser wenig löslichen Wirkstoffe zu verstehen. Als Beispiele für derartige Wirkstoffe seien im einzelnen genannt :

2-Isopropoxy-phenyl-N-methylcarbamat
O,O-Diethyl-O-(4-nitro-phenyl)-thionophosphorsäureester
O,O-Dimethyl-O-(4-nitro-phenyl)-thionophosphorsäureester
S-[1,2-Bis-(ethoxycarbonyl)-ethyl]-O,O-dimethyl-dithiophosphorsäureester
O,O-Dimethyl-O-(3-methyl-4-nitro-phenyl)-thionophosphorsäureester
O,O-Dimethyl-O-(4-methylmercapto-3-methyl-phenyl)-thionophosphorsäureester
γ-Hexachlorcyclohexan
(Cyclohex-1-en-1,2-dicarboximidomethyl)-2,2-dimethyl-3-(2-methylpropenyl)-cyclopropancarboxylat

Unter pharmakologisch wirksamen Stoffen sind im vorliegenden Fall vorzugsweise in veterinärmedizinischen Bereich einsetzbare, in Wasser wenig lösliche Stoffe zu verstehen. Als Beispiel für derartige Wirkstoffe sei genannt :

2,2-Dimethyl-3-[β-(p-chlorphenyl)-β-chlorvinyl]-cyclopropancarbonsäure-α-cyano-3-phenoxy-4-fluor-benzylester

Die in den erfindungsgemäßen Öl-in-Wasser-Emulsionen als Emulgatoren enthaltenen Alkylaryl-polyglykolether sind durch die Formel (I) definiert. Die Zahlen für den Index n stellen Durchschnittswerte dar.

Als Beispiele für Alkylaryl-polyglykolether der Formel (I) seien im einzelnen genannt :

Bis-[α-methyl-(4-methyl-benzyl)]-phenyl-polyglykolether mit durchschnittlich 27 Oxyethylen-Einheiten pro Molekül.

Bis-[α-methyl-(4-n-dodecyl-benzyl)]-phenyl-polyglykolether mit durchschnittlich 27 Oxyethylen-Einheiten pro Molekül.

Tris-[α-methyl-(4-methyl-benzyl)]-phenyl-polyglykolether mit durchschnittlich 17 Oxyethylen-Einheiten pro Molekül.

Die in der Praxis verwendeten Emulgatoren dieses Typs sind im allgemeinen Gemische aus mehreren Verbindungen der Formel (I). Insbesondere handelt es sich hierbei um Gemische aus Stoffen der Formel (I), die sich durch den Substitutionsgrad an dem mit der Ethylenoxid-Einheit verbundenen Phenylring unterscheiden. Dadurch errechnen sich für den Index m auch gebrochene Zahlen als Mittelwerte. Beispielsweise erwähnt seien Substanzen, für die sich folgende durchschnittliche Zusammensetzungen ergeben :

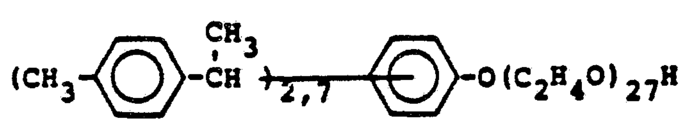

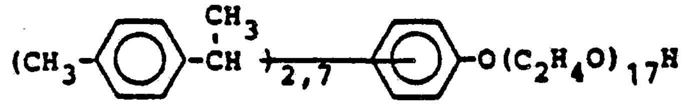

Die Alkylaryl-polyglykolether der Formel (I) sind bekannt.

Als Beispiele für Alkylaryl-sulfonsäure-Salze der Formel (II) seien im einzelnen genannt:

4-(n-Nonyl)-phenyl-sulfonsäure-Natriumsalz
4-(n-Dodecyl)-phenyl-sulfonsäure-Calciumsalz
4-(Tetrapropylen)-phenyl-sulfonsäure-Natriumsalz
4-(n-Nonyl)-phenyl-sulfonsäure-Calciumsalz

Die Alkylaryl-sulfonsäure-Salze der Formel (II) sind bekannt. Sie werden im allgemeinen als 50-75 %ige Lösungen in organischen Lösungsmitteln, z. B. n- oder i-Butanol, eingesetzt, können grundsätzlich oder auch ohne Lösungsmittel verwendet werden.

Als organische Lösungsmittel, die in den erfindungsgemäßen Öl-in-Wasser-Emulsionen gegebenenfalls enthalten sein können, kommen alle üblichen, mit Wasser wenig mischbaren organischen Solventien in Frage. Vorzugsweise genannt seien aromatische Kohlenwasserstoffe, wie Xylol, Toluol und Dimethyl-naphthalin, ferner chlorierte aromatische Kohlenwasserstoffe, wie Chlorbenzole, außerdem aliphatische Kohlenwasserstoffe, wie Benzin und Petrolether, weiterhin halogenierte aliphatische Kohlenwasserstoffe, wie Methylenchlorid und Chloroform, darüber hinaus cycloaliphatische Kohlenwasserstoffe, wie Cyclohexan, außerdem Alkohole und Ketone, wie n-Butanol, n-Hexanol, iso-Hexanol, n-Octanol, Cyclohexanol, Benzylalkohol, Di-n-butyl-keton und Isophoron, und ferner Ether und Ester, wie Glykolmonomethylether und Glykolmonomethyletheracetat.

Als Solubilisatoren, die in den erfindungsgemäßen Öl-in-Wasser-Emulsionen enthalten sein können, kommen alle üblichen Lösungsvermittler in Betracht. Vorzugsweise verwendbar sind Alkylphenole oder Kresole, die pro Mol mit 1 bis 8 Mol Ethylenoxid kondensiert sind. Speziell genannt sei in diesem Zusammenhang p-Kresol, das pro Mol mit 1 bis 8 Mol Ethylenoxid kondensiert ist.

Als Zusatzstoffe, die in den erfindungsgemäßen Öl-in-Wasser-Emulsionen gegebenenfalls enthalten sein können, kommen Konservierungsmittel, Farbstoffe und Kältestabilisatoren in Betracht.

Als Beispiele für Konservierungsmittel seien 2-Hydroxybiphenyl und Sorbinsäure genannt. Als Beispiele für Farbstoffe seien Azofarbstoffe und Phthalocyanin-Farbstoffe angeführt. Als Beispiele für Kältestabilisatoren seien Harnstoff, Zucker und Salze, wie Ammoniumsulfat und Natriumoleat genannt. Als Beispiel für einen Synergisten sei 3,4-Methylendioxy-6-propyl-benzyl-n-butyl-diethylenglykolether (Piperonylbutoxid) genannt.

In den erfindungsgemäßen Öl-in-Wasser-Emulsionen kann das Verhältnis von Wirkstoff(en) gegebenenfalls in Gemisch mit organischen Solventien und/oder Solubilisatoren einerseits zu Alkylaryl-polyglykolether im Gemisch mit Alkyl-aryl-sulfonsäure-Salz andererseits in einem bestimmten Bereich variiert werden. Im allgemeinen entfallen auf 1 Teil Emulgatorgemisch 1 bis 8 Gew.-Teile, vorzugsweise 2 bis 6 Gew.-Teile an Wirkstoff(en) gegebenenfalls im Gemisch mit organischen Solventien und/oder Solubilisatoren.

Bei der Herstellung der erfindungsgemäßen Öl-in-Wasser-Emulsionen können vorzugsweise alle diejenigen Komponenten verwendet werden, die bereits im Zusammenhang mit der Beschreibung der erfindungsgemäßen Öl-in-Wasser-Emulsionen vorzugsweise genannt wurden.

Verwendet man bei dem erfindungsgemäßen Verfahren einen Wirkstoff, der bei Temperaturen bis zu 40 °C im flüssigen Zustand vorliegt, so erübrigt sich im allgemeinen die Zugabe eines mit Wasser wenig mischbaren organischen Lösungsmittels und/oder eines Solubilisators.

Verwendet man bei dem erfindungsgemäßen Verfahren hingegen einen Wirkstoff, der bei Temperaturen bis zu 40 °C im festen Zustand vorliegt, so ist es erforderlich, den betreffenden Wirkstoff vor der Emulgierung in einem mit Wasser wenig mischbaren organischen Lösungsmittel und/oder Solubilisator zu lösen. Die Menge an organischem Solvens und/oder Solubilisator wird dabei so bemessen, daß sie gerade zur Lösung der Festsubstanz ausreicht. Die Reaktionstemperaturen können bei dem erfindungsgemäßen Verfahren innerhalb eines größeren Bereiches variiert werden. Im allgemeinen arbeitet man bei Temperaturen zwischen 10 °C und 80 °C, vorzugsweise zwischen 20 °C und 60 °C.

Bei der Durchführung des erfindungsgemäßen Verfahrens geht man im allgemeinen so vor, daß man zunächst eine homogene Lösung aus einem oder mehreren Wirkstoffen, Alkylarylpolyglykolether und Alkylarylsulfonsäure-Salz, gegebenenfalls mit Wasser wenig mischbarem organischen Lösungsmittel und/oder Solubilisator sowie gegebenenfalls Zusatzstoffen herstellt und diese Mischung dann unter Rühren in gegebenenfalls Zusatzstoffe enthaltendes Wasser gibt. Die Mengen der Komponenten werden dabei so gewählt, daß die Komponenten in der resultierenden Öl-in-Wasser-Emulsion in der jeweils gewünschten Konzentration vorliegen. Die Reihenfolge, in der die Komponenten der organischen Phase zusammengegeben werden ist variabel. Die Zugabe der organischen Phase zu der wäßrigen Phase erfolgt zweckmäßigerweise langsam unter gleichmäßigem Rühren mit üblichen Rührgeräten. Dabei bildet sich eine feinteilige Mikroemulsion, in der die Tröpfchen einen Durchmesser zwischen 0,05 µm und 1 µm aufweisen. Bei schneller Zugabe der organischen Phase zur wäßrigen Phase ist das Spektrum der Tröpfchengrößen breiter und zur Seite der Tröpfchen mit größerem Durchmesser verschoben. Eine Nachbehandlung der entstehenden Öl-in-Wasser-Emulsion mit Homogenisatoren ist nicht notwendig, kann aber vorgenommen werden, sofern dieses gewünscht wird.

Die erfindungsgemäßen Öl-in-Wasser-Emulsionen können entweder in der zubereiteten Form oder nach vorheriger Verdünnung appliziert werden. Die Aufwandmenge richtet sich dabei nach der Konzentration der Öl-in-Wasser-Emulsion und nach der jeweiligen Indikation.

Die Anwendung der erfindungsgemäßen Öl-in-Wasser-Emulsionen erfolgt nach den üblichen Methoden, also zum Beispiel durch Spritzen, Sprühen oder Gießen.

Die Herstellung der erfindungsgemäßen Öl-in-Wasser-Emulsionen geht aus den folgenden Beispielen hervor.

## Herstellungsbeispiele

### Beispiel 1

25,0 g des insektiziden Wirkstoffes O,O-Dimethyl-O-(4-methylmercapto-3-methyl-phenyl)-thionophosphorsäureester werden unter Rühren bei Temperaturen zwischen 20 °C und 40 °C mit 10,0 g eines Emulgatorgemisches versetzt, das zu gleichen Teilen aus den Komponenten besteht, die sich formelmäßig wie folgt beschreiben lassen :

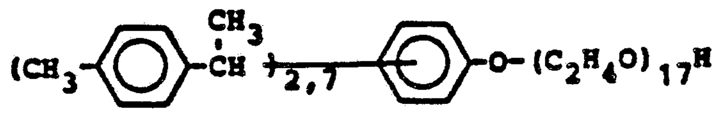

(I-1)

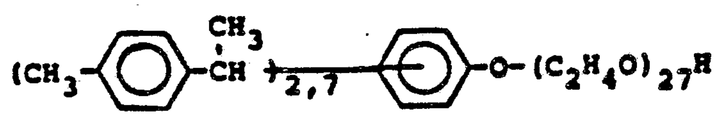

(I-2)

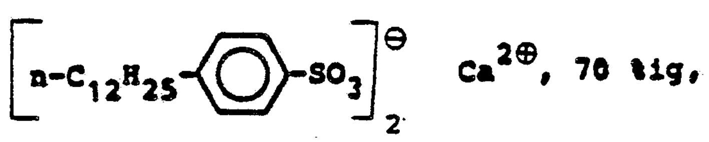

(II-1)

gelöst in n-Butanol

Die dabei entstehende homogene Lösung wird innerhalb von 2 Minuten in eine mit einem Flügelrührer mit einer Geschwindigkeit von 2 000 Umdrehungen pro Minute gerührte Lösung von 0,2 g 2-Hydroxy-biphenyl in 64,8 g destilliertem Wasser gegeben. Nach beendeter Zugabe wird noch 5 Minuten nachgerührt. Es bildet sich eine rötlich aussehende, viskose, kolloidale Emulsion, die bei zwanzigwöchiger Lagerung bei Temperaturen unterhalb von 35 °C keine physikalischen oder chemischen Veränderungen zeigt.

### Beispiel 2

50 g O,O-Dimethyl-O-(4-methylmercapto-3-methyl-phenyl)-thionophosphorsäureester werden unter Rühren bei Temperaturen zwischen 20 und 40 °C mit 12 g eines Emulgatorgemisches versetzt, das zu 80 Gew.-% aus dem Alkylaryl-polyglykolether der Formel (I-1) und zu 20 Gew.-% aus 4(n-Dodecyl)-phenylsulfonsäure-Calciumsalz der Formel (II-1) (in Form einer 70 %igen Lösung in n-Butanol) besteht. Die dabei entstehende homogene Lösung wird innerhalb von 3 Minuten in eine mit einem Flügelrührer mit einer Geschwindigkeit von 2 000 Umdrehungen pro Minute gerührte Lösung von 0,2 g 2-Hydroxy-biphenyl in 37,8 g destilliertem Wasser gegeben. Es bildet sich eine rötlich aussehende hochviskose, kolloidale Emulsion, die bei zwanzigwöchiger Lagerung bei Temperaturen unterhalb von 35 °C keine physikalischen oder chemischen Veränderungen zeigt.

### Beispiel 3

25,0 g O-Ethyl-O-(3-methyl-4-methylthiophenyl)-N-isopropyl-phosphoramidat werden mit 20,0 g eines Solubilisators, der durch Kondensation von 1 Mol p-Kresol mit 1,5-2 Mol Ethylenoxid hergestellt wurde, versetzt. Man rührt bei Raumtemperatur bis zur vollständigen Lösung des Feststoffes und fügt dann nacheinander 2,0 g eines zu gleichen Teilen aus Stoffen der Formeln (I-1), (I-2) und (II-1) bestehenden Emulgatorgemisches und 10,0 g eines Emulgators der Formel (I-1) hinzu. Die dabei entstehende homogene Lösung wird innerhalb von 2 Minuten in eine mit einem Flügelrührer mit einer Geschwindigkeit von 2 000 Umdrehungen pro Minute gerührte Lösung von 0,2 g 2-Hydroxy-biphenyl in 42,8 g destilliertem Wasser gegeben. Nach beendeter Zugabe wird noch 6 Minuten nachgerührt. Es bildet sich eine leicht gelbliche viskose, kolloidale Emulsion, die bei Lagerung bei Temperaturen unterhalb von 35 °C auch nach Wochen keine physikalische oder chemische Veränderung zeigt.

Beispiel 4

Nach der im Beispiel 1 angegebenen Methode wird eine organische Phase aus 25,0 g O-Ethyl-S-propyl-O-(4-methylmercapto-phenyl)-thionophosphorsäureester und 10,0 g eines Emulgators der Formel (I-1) in eine wäßrige Phase aus 0,2 g 2-Hydroxy-biphenyl und 64,8 g destilliertem Wasser eingerührt. Es bildet sich eine wenig viskose, weiße Emulsion, die bei zwanzigwöchiger Lagerung bei Temperaturen bis zu 40 °C keine physikalische oder chemische Veränderung zeigt.

Beispiel 5

Nach der im Beispiel 3 angegebenen Methode wird eine organische Phase aus 1 g 2-Isopropoxy-phenyl-N-methyl-carbamat, 0,2 g (Cyclohex-1-en-1,2-dicarboximidomethyl)-2,2-dimethyl-3-(2-methylpropenyl)-cyclopropancarboxylat 1,0 g 3,4-Methylendioxy-6-propylbenzyl-N-butyldiethylenglykolether, 5,0 g n-Hexanol, 5,0 g Xylol, 6,0 g Kerosen und 5,0 g eines Emulgatorgemisches aus 80 Gew.-% einer Verbindung der Formel (I-1) und 20 Gew.-% einer Verbindung der Formel (II-1) in 76,8 g destilliertes Wasser eingerührt. Es bildet sich eine gelblich-weiße Mikroemulsion, die nach achtwöchiger Lagerung bei 54 °C keine physikalische oder chemische Veränderung zeigt.

Beispiel 6

Nach der im Beispiel 1 angegebenen Methode wird eine organische Phase aus 25,0 g des insektiziden Wirkstoffes O,O-Dimethyl-O-(4-methylmercapto-3-methyl-phenyl)-thionophosphorsäureester und 10,0 g des im Beispiel 1 genannten Emulgators in 65,0 g destilliertes Wasser eingerührt. Es bildet sich eine rötlich aussehende, viskose, kolloidale Emulsion, die bei zwanzigwöchiger Lagerung bei Temperaturen unterhalb von 35 °C keine physikalischen oder chemischen Veränderungen zeigt.

Beispiel 7

Nach der im Beispiel 1 angegebenen Methode wird eine organische Phase aus 25,0 g des insektiziden Wirkstoffes O,O-Dimethyl-O-(4-methylmercapto-3-methyl-phenyl)-thionophosphorsäureester, 7,0 g des im Beispiel 1 genannten Emulgators und 0,7 g Benzylalkohol in 67,3 g destilliertes Wasser eingerührt. Es bildet sich eine rötlich aussehende, viskose, kolloidale Emulsion, die bei zwanzigwöchiger Lagerung bei Temperaturen unterhalb von 35 °C keine physikalischen oder chemischen Veränderungen zeigt.

Beispiel 8

Nach der im Beispiel 3 angegebenen Methode wird eine organische Phase aus 5,0 g 2,2-Dimethyl-3-(β,β-dichlorvinyl)-cyclopropancarbonsäure-α-cyano-3-phenoxy-4-fluor-benzylester, 10,0 g Xylol und 8,0 g eines Emulgators, der aus 48 Gew.-% einer Verbindung der Formel (I-2), 32 Gew.-% einer Verbindung der Formel (II-1), 16 Gew.-% n-Butanol und 4 Gew.-% Xylol besteht, in 77,0 g destilliertes Wasser eingerührt. Es bildet sich eine Mikroemulsion, die bei zwanzigwöchiger Lagerung bei Temperaturen unterhalb von 35 °C keine physikalischen oder chemischen Veränderungen zeigt.

Beispiel 9

Nach der im Beispiel 3 angegebenen Methode wird eine organische Phase aus 5,0 g 2,2-Dimethyl-3-(β,β-dichlorvinyl)-cyclopropancarbonsäure-α-cyano-3-phenoxy-4-fluor-benzylester, 10,0 g Xylol, 2,0 g n-Butanol und 5,0 g eines Emulgators, der aus 48 Gew.-% einer Verbindung der Formel (I-2), 32 Gew.-% einer Verbindung der Formel (II-1), 16 Gew.-% n-Butanol und 4 Gew.-% Xylol besteht, in 78,0 g destilliertes Wasser eingerührt. Es bildet sich eine Mikroemulsion, die bei zwanzigwöchiger Lagerung bei Temperaturen unterhalb von 35 °C keine physikalischen oder chemischen Veränderungen zeigt.

**Patentansprüche**

1. Öl-in-Wasser-Emulsionen, gekennzeichnet durch einen Gehalt von

— 5 bis 80 Gew.-% mindestens eines in Wasser wenig löslichen agrochemischen Wirkstoffes, eines Wirkstoffes zur Bekämpfung von Schädlingen im Haushalts- und Hygiene-Bereich und/oder eines Pharmakologisch wirksamen Stoffes,

— 3 bis 15 Gew.-% mindestens eines Alkylarylpolyglykolethers der Formel

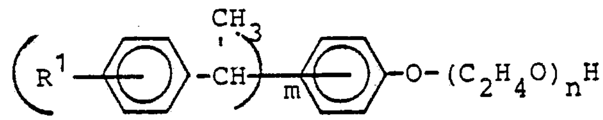

(I)

in welcher
$R^1$ für Wasserstoff oder Methyl steht,
m für 2 oder 3 steht und
n für ganze Zahlen von 12 bis 40 steht,
im Gemisch mit Alkylarylsulfonsäure-Salzen der Formel

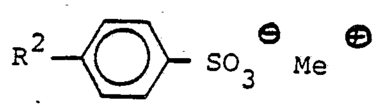

(II)

in welcher
$R^2$ für Alkyl mit 9 bis 30 Kohlenstoffatomen steht und
$Me^{\oplus}$ für ein Natriumkation oder für ein Äquivalent eines Calciumkations steht,
— und Wasser, und
— gegebenenfalls 5 bis 20 Gew.-% mindestens eines mit Wasser wenig mischbaren organischen Lösungsmittels und/oder eines Solubilisators sowie
— gegebenenfalls 0,1 bis 10 Gew.-% an Zusatzstoffen,
wobei die Summe der Komponenten jeweils 100 Gew.-% beträgt.

2. Verfahren zur Herstellung von Öl-in Wasser-Emulsionen gemäß Anspruch 1, dadurch gekennzeichnet, daß man eine homogene Mischung aus
— mindestens einem in Wasser wenig löslichen agrochemischen Wirkstoff einem Wirkstoff zur Bekämpfung von Schädlingen im Haushalts- und Hygiene-Bereich und/oder einem pharmakologisch wirksamen Stoff,
— mindestens einem Alkylaryl-polyglykolether der Formel

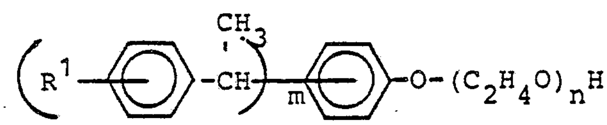

(I)

in welcher
$R^1$, m und n die oben angegebene Bedeutung haben, im Gemisch mit Alkylaryl-sulfonsäure-Salzen der Formel

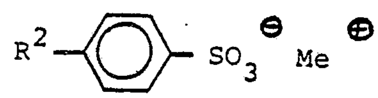

(II)

in welcher
$R^2$ und $Me^{\oplus}$ die oben angegebene Bedeutung haben,
— gegebenenfalls mindestens einem mit Wasser wenig mischbaren organischen Lösungsmittel und/oder eines Solubilisators sowie
— gegebenenfalls Zusatzstoffen
unter Rühren in gegebenenfalls Zusatzstoffe enthaltendes Wasser gibt.

3. Verwendung von Öl-in-Wasser-Emulsionen gemäß Anspruch 1 in der Landwirtschaft und im Gartenbau, im Haushalts- und Hygiene-Bereich und/oder im medizinischen Bereich, wobei die Verwendung in Verfahren gemäß Artikel 52 (4) EPÜ ausgenommen ist.

**Claims**

1. Oil-in-water emulsions, characterised in that they contain
— 5 to 80 % by weight of at least one sparingly water-soluble agrochemical active compound, at least one active compound for combating pests in the domestic field and hygiene field and/or at least one pharmacologically active compound,
— 3 to 15 % by weight of at least one alkylaryl polyglycol ether of the formula

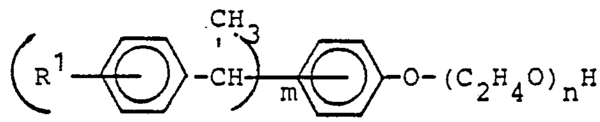

(I)

in which

$R^1$ represents hydrogen or methyl,

m represents 2 or 3 and

n represents integers from 12 to 40,

in admixture with alkylarylsulphonic acid salts of the formula

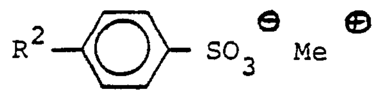

(II)

in which

$R^2$ represents alkyl with 9 to 30 carbon atoms and

$Me^{\oplus}$ represents a sodium cation or an equivalent of a calcium cation,

— and water, and

— if necessary, 5 to 20 % by weight of at least one poorly watermiscible organic solvent and/or at least one solubiliser, and

— if appropriate, 0.1 to 10 % by weight of additives,

the sum of the components being in each case 100 % by weight.

2. Process for the preparation of oil-in-water emulsions according to Claim 1, characterised in that a homogeneous mixture of

— at least one sparingly water-soluble agrochemical active compound, at least one active compound for combating pests in the domestic field and hygiene field and/or at least one pharmacologically active compound,

— at least one alkylaryl polyglycol ether of the formula

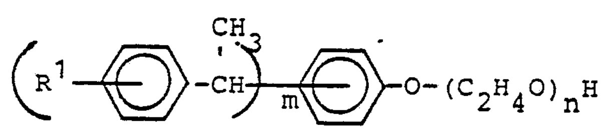

(I)

in which

$R^1$, m and n have the meaning indicated above, in admixture with alkylarylsulphonic acid salts of the formula

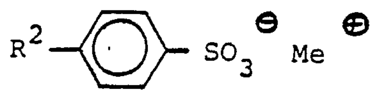

(II)

in which

$R^2$ and $Me^{\oplus}$ have the meaning indicated above ;

— if necessary, at least one poorly water-miscible organic solvent and/or at least one solubiliser and

— if appropriate, additives,

is added, with stirring, to water which, if appropriate, contains additives.

3. Use of oil-in-water emulsions according to Claim 1 in agriculture and in horticulture, in the domestic field and hygiene field and/or in the medical field, with the exception of the use in procedures according to Article 52 (4) of the EPC.

**Revendications**

1. Emulsions huile-dans-eau, caractérisées par une teneur de

— 5 à 80 % en poids d'au moins une substance active agrochimique peu soluble dans l'eau, d'une substance active pour combattre des parasites dans le secteur ménager et le secteur de l'hygiène et/ou d'une substance douée d'activité pharmacologique,

— 3 à 15 % en poids d'au moins un éther d'alkylarylpolyglycol de formule

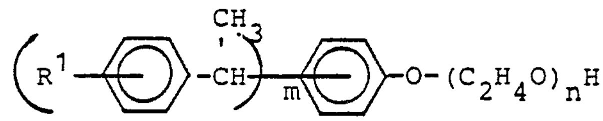

(I)

dans laquelle

$R^1$ représente l'hydrogène ou le groupe méthyle,

m a la valeur 2 ou 3 et

n représente des nombres entiers de 12 à 40,

en mélange avec des sels d'acides alkylarylsulfoniques de formule

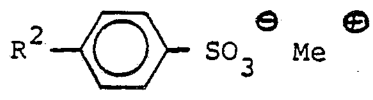 (II)

dans laquelle

$R^2$ est un groupe alkyle ayant 9 à 30 atomes de carbone et

$Me^{\oplus}$ représente un cation sodium ou est un équivalent d'un cation calcium,

— et de l'eau, et

— le cas échéant 5 à 20 % en poids d'au moins un solvant organique peu miscible à l'eau et/ou d'un agent solubilisant, ainsi que

— le cas échéant 0,1 à 10 % en poids d'additifs,

la somme des composants s'élevant dans chaque cas à 100 % en poids.

2. Procédé de production d'émulsions huile-dans-eau suivant la revendication 1, caractérisé en ce qu'on verse sous agitation dans de l'eau contenant éventuellement des additifs, un mélange homogène formé

— d'au moins une substance active agrochimique peu soluble dans l'eau, une substance active pour combattre des parasites dans le secteur ménager et le secteur de l'hygiène et/ou une substance douée d'activité pharmacologique,

— d'au moins un éther d'alkylarylpolyglycol de formule

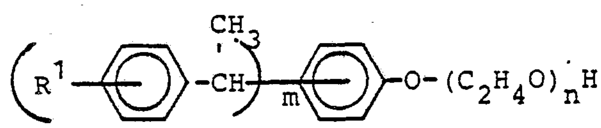 (I)

dans laquelle

$R^1$, m et n ont les définitions indiquées ci-dessus,

en mélange avec des sels d'acides alkylarylsulfoniques de formule

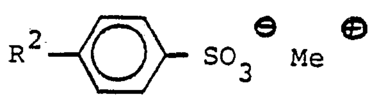 (II)

dans laquelle

$R^2$ et $Me^{\oplus}$ ont la définition indiquée ci-dessus,

— le cas échéant d'au moins un solvant organique peu miscible à l'eau et/ou un agent solubilisant ainsi que

— le cas échéant des additifs.

3. Utilisation d'émulsions huile-dans-eau suivant la revendication 1 en agriculture et en horticulture, dans le secteur ménager et dans le secteur de l'hygiène et/ou dans le secteur médical, cette utilisation dans le procédé suivant l'article 52 (4) EPÜ étant exclue.